(19) Europäisches Patentamt
European Patent Office
Office européen des brevets

(11) EP 1 262 544 A2

(12) **EUROPEAN PATENT APPLICATION**
published in accordance with Art. 158(3) EPC

(43) Date of publication:
04.12.2002 Bulletin 2002/49

(51) Int Cl.$^7$: **C12N 15/00**, C07B 61/00,
B01J 19/00

(21) Application number: 00980068.1

(22) Date of filing: 15.11.2000

(86) International application number:
PCT/MX00/00047

(87) International publication number:
WO 01/036439 (25.05.2001 Gazette 2001/21)

(84) Designated Contracting States:
AT BE CH CY DE DK ES FI FR GB GR IE IT LI LU MC NL PT SE TR
Designated Extension States:
AL LT LV MK RO SI

(30) Priority: 15.11.1999 MX 9910476

(71) Applicant: UNIVERSIDAD NACIONAL
AUTONOMA DE MEXICO
Mexico 04510 D.F. (MX)

(72) Inventors:
• SOBERON MAINERO, Francisco Xavier
  Cuernavaca, Morelos 62330 (MX)
• GAYTAN COLIN, Rubén Paúl
  Cuernavaca, Morelos 62130 (MX)

(74) Representative:
Van Someren, Petronella F. H. M. et al
Arnold & Siedsma,
Advocaten en Octrooigemachtigden,
Sweelinckplein 1
2517 GK Den Haag (NL)

(54) **METHOD FOR CONSTRUCTING OLIGODESOXYRIBONUCLEOTIDE BINOMIAL LIBRARIES WITH CODON MUTAGENIZED USING DESOXYNUCLEOSIDE-PHOSPHORAMITIDES**

(57) The invention refers to a mutagenesis method for the construction of binomial libraries of oligodeoxyribonucleotides mutagenized at a codon level that comprises the use of two sets of deoxynucleoside-phosphoramidites protected in the 5' hydroxyl by means of protecting groups orthogonal to one another, which are combined during oligodeoxyribonucleotide synthesis.

This method constitutes a valuable tool for- the study of the structure-function relationship of proteins and protein engineering, since it is possible to generate in a controlled, predictable way few replacements of amino acids per protein. This permits research into the individual importance of each of the wild-type amino acids to the function of the protein and, at the same time, avoids their functional destruction. The purpose is to improve a countless number of proteins with commercial application, like for example the enzymes called subtilisins that are used in biodegradable detergents.

Figure 2

EP 1 262 544 A2

**Description**

TECHNICAL FIELD OF THE INVENTION

The invention described in this document refers to a mutagenesis method for the construction of libraries of oligodeoxyribonucleotides mutagenized at a codon level, by means of which it is possible to control the generation of binomial distributions of mutants to few or many replacements of codons per mutant oligonucleotide in a directly proportional way to the level of mutagenesis. This method comprises the use of two sets of deoxyribonucleoside-phosphoramidites (dNP's) protected in their 5' hydroxyl by the orthogonal protecting groups, 4,4'- dimethoxytrityl (DMT) or 9-fluorenylmethoxycarbonyl (Fmoc), which are combined during automated oligodeoxyribonucleotide synthesis.

BACKGROUND TO THE INVENTION

Proteins are biological polymers mainly composed of 20 different amino acids. At cellular level they are responsible for the control of many vital functions as for example the enzymatic synthesis of compounds, regulation of biological processes, etc. Each of the amino acids in a protein is encoded by at least one group of three deoxyribonucleotides called triplet or codon.

At present it is known that the function of a protein, such as molecular recognition or catalysis, depends on its characteristic three-dimensional structure, ordered by its primary amino acid sequence. For this reason, many research groups are interested in studying the structure-function relationship of proteins in order to rationally improve a large amount of enzymes that could well have a commercial application.

Protein engineering is the structural modification of these materials through the modification of their encoding gene with the purpose of understanding the relationships established between the amino acid sequence, the final folding of the polypeptide chain and its function.

Such a work is feasible thanks to the advances in recombinant DNA technology and DNA chemical synthesis methods, that make possible to substitute, delete or insert any amino acid in a protein, by modifying the respective codon in its encoding gene. It is known that a large number of proteins can dramatically alter their properties due to a change in just one functionally critical amino acid.

Mutagenesis directed at one specific site (by nucleotide or codon) through synthetic oligodeoxyribonucleotides is being extensively used in almost all the biochemical disciplines, in order to explore the relationship between the structure and function of proteins. However, the selective perturbation of individual amino acids requires some understanding at a molecular level, of the interacting structures (protein-protein, enzyme-substrate or protein-DNA). That is, it is necessary to have some bases in order to predict the changes in some amino acid residues that will produce a particular functional consequence in the protein. Therefore, rational attempts in protein engineering and evolution to modify already existing proteins are limited by the need to have a high resolution structure of the protein of interest and a good understanding of its molecular mechanism. Unfortunately, there are few systems that fulfill this requirement [Hermes, J.D., 1989].

One alternative method, of a more general character, for both understanding the structure-function relationships and for protein engineering, uses random mutagenesis, particularly one of its versions so-called combinatorial mutagenesis. In this method a "mixture" of random mutants (library) is generated instead of introducing particular premeditated changes. Once the mutant gene library has been created, the genes are cloned and the proteins that these genes encode are expressed in an appropriate host. The transformed colonies are then selected or screened in the search for the appearance of a phenotype caused by the properties of the new proteins (e.g. greater thermostability, greater catalytic rate, different specificity, etc.). When the procedure permits to explore different combinations of mutants, it is called combinatorial mutagenesis.

A large number of protocols for random mutagenesis of genes have been described in the literature and, in general, they can be classified as chemical methods [Botstein, D., 1985], enzymatic methods [Lehtovaara, P.M., 1988] and oligo-directed methods [Hermes, J.D., 1989].

The chemical methods require exposure of the microorganism to mutagenic reagents [Myers, R.M., 1985 and Kadonaga, J.T., 1985], such as sodium bisulphite, hydroxylamine and nitrous acid, among others, which cause modifications to the deoxynucleotides that comprise cellular DNA. In the enzymatic methods [Lehtovara, et al., 1988 and Reeve M.A., 1995] it is necessary to make use of polymerases that in certain experimental conditions make mistakes during the addition of nucleotides. Some examples of these enzymes are the reverse transcriptase of myeloblastosis virus and the Taq polymerase. In both cases, libraries of mutant genes that contain punctual changes in nucleotides are mainly generated, making only possible to analyze from 18% to 40% of all possible changes in the amino acids as a direct consequence of the degeneracy of the genetic code [Sirotkin, K., 1986]. In both methods, the mutagenesis window, the amino acid distribution and the mutagenesis level are difficult to control.

The combinatorial mutagenesis method directed by oligonucleotides resorts to the use of synthetic oligodeoxyribonucleotide libraries, produced in just one experiment by means of mixtures of the four deoxynucleotides [Dunn I.S., 1988 and Del Rio, G., 1994]. This type of mutagenesis can be performed by "saturation" or "contamination". In the first method, each wild-type deoxynucleotide of the codons to be mutated is substituted by a mixture of the four deoxynucleotides, thus generating a library of variant codons $64^n$ in size ($32^n$ when using the NNG/C system), where n represents the number of codons to be substituted. Considering a practical transformation efficiency of $10^7$ to $10^9$ colonies per oligodeoxyribonucleotide library, with this methodology it is only possible to analyze a maximum of 5 amino acids per experiment. However, the main drawback of this methodology is the generation of high multiplicity mutants (several codon changes per gene) which normally produce a loss in protein function.

Combinatorial mutagenesis by contamination, directed by oligodeoxyribonucleotides, consists in contaminating each of the wild-type nucleotidic couplings during the chemical synthesis of the oligonucleotide with a small proportion ($\alpha$) of the 3 non wild-type deoxynucleotides [Hermes, J.D., 1989 and Ner S.S., 1988], Although this methodology makes possible to obtain libraries enriched with low multiplicity mutants (few changes in codons per gene) and explore relatively large mutagenesis windows, the problem inherent to the degeneracy of the genetic code is still present; that is, the substitution of those amino acids whose codons vary on one base with respect to the wild-type ones is still favored [Sirotkin K., 1986].

The ideal protocol for random mutagenesis must cover many requirements. First, the amino acids region to be explored (that is, the mutagenesis window) must be easily specified. Second, each codon located in the mutagenesis window must have the same probability of being substituted for (homogeneous distribution). Third, the substitution of one amino acid for any of the other 19 must take place with the same probability (homogeneous frequency). Fourth, the system must permit the definition of immutable positions within the mutagenesis window. Fifth, it must be possible to control the rate or level of mutagenesis ($\alpha$) in order to adjust the density of the mutants desired through the theory of combinatorial analysis; and sixth, mutagenesis efficiency must be high so that the majority of the clones analyzed correspond to mutant sequences [Hermes, J.D., 1989; Lehtovaara, P.M., 1988 and Sondek, J. & Shortle, D., 1992].

Some authors agree that the ideal combinatorial mutagenesis method must involve the use of mixtures of trinucleotides [Vinerkäs et al., 1994; Ono et al., 1995 and Kayushin et al., 1996] in order to generate mutagenesis at a codon level and not at a nucleotide level so that the space of sequences can be explored in a better way. It must be possible to couple these trinucleotides in a substoichiometric manner during the conventional synthesis of oligodeoxyribonucleotides in order to generate libraries that follow a predictable binomial distribution of mutants. The method mentioned above would avoid the problems associated with the degeneracy of the genetic code and would permit a homogeneous distribution and frequency of variants. These reagents, however, are not yet commercially available and their preparation involves considerable time and cost (U:S: Serial No. 60/123,438).

Another of the methods used to perform codon-based mutagenesis is the so-called "resin-splitting method", (Cormack, B.P., 1993, Glaser, S.M., 1992, Hooft van Huijsduijnen, R.A.M., 1992) in which the oligonucleotide is synthesized in a column or reactor to the point where it is wished for mutagenesis to begin. The column is then dismantled in order to begin codon substitution. The CPG support containing the growing oligonucleotide is separated in two portions (according to the level of mutagenesis defined by the size of the window and the multiplicity of substitutions to be favored), which are repacked in one column called 'mutant column' and another column called 'wild-type column'. The mutant column is subject to three cycles of synthesis with an equimolar mixture of the four deoxynucleoside-phosphoramidites (N) in the first two positions of the codon and an equimolar mixture of G/C in the third position to generate an NNG/C combination that produces 32 codons that encode at least once for each of the twenty amino acids. The wild-type column is submitted to three cycles of synthesis with the dNP's that define the wild-type sequence. The two columns are reopened, the support of both is combined and separated again repeating the process for each codon to be mutated.

In spite of the apparent simplicity of the resin-splitting method, it is really very laborious and tedious. It requires large amounts of CPG support and therefore large amount of dNP's in order to be able to handle low levels of mutagenesis and relatively large mutagenesis windows; otherwise it can only be applied for high levels of mutagenesis and small mutagenesis windows.

DETAILED DESCRIPTION OF THE INVENTION

BRIEF DESCRIPTION OF THE FIGURES

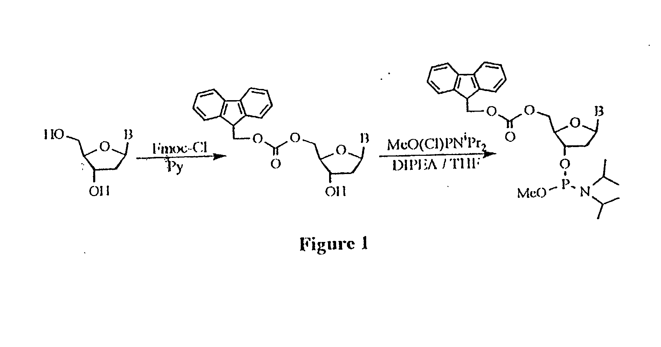

Figure 1. Illustrates the synthesis of Fmoc-deoxyribonucleosides and their corresponding methyl-phosphoramidites. B = 6N-benzoyladenine, 4N-benzoylcytosine, 2N-isobutyrylguanine or thymine. Fmoc-Cl = 9-fluorenylmethoxycarbonyl chloride, Py = pyridine, DIPEA = N,N-diisopropylethylamine.

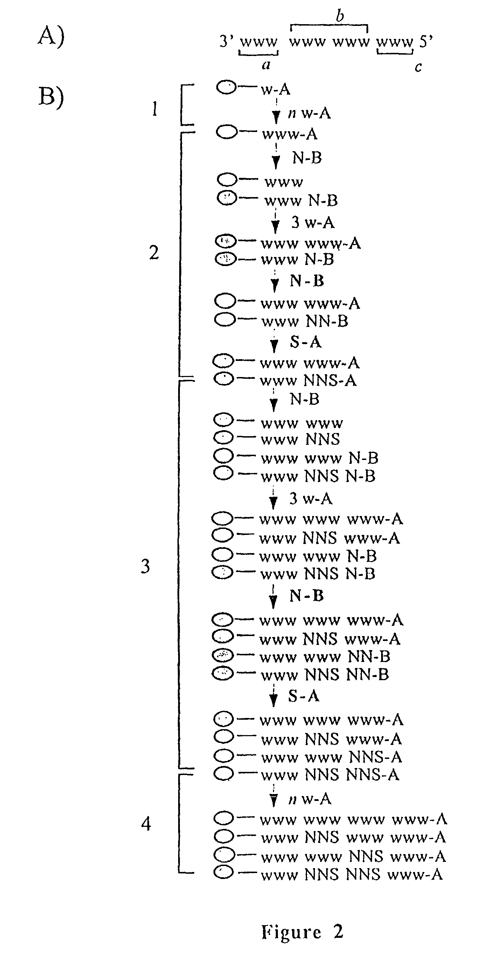

Figure 2. Protocol, herein called "pre-addition" protocol, for the generation of oligodeoxyribonucleotide libraries using the Orthogonal Combinatorial Mutagenesis method (OCM). A) Exemplified Wild-type sequence to be mutated. This sequence comprises: a) the 3' adjacent region, b) mutagenesis window including as example only two amino acids and c) the 5' adjacent region. B) Procedure for the automatic assembly of oligodeoxyribonucleotide libraries using the pre-addition protocol.1) assembly of the wild-type sequence of the 3' adjacent region with the appropriate A-dNP's; 2) first mutagenesis cycle with an ordered combination of B-dNP's and A-dNP's; 3) second mutagenesis cycle and 4) assembly of the wild-type sequence of the 5' adjacent region with A-dNP's. Where: ⊛ - Represents the CPG support, w = wild-type deoxyribonucleotide, A-dNP = deoxyribonucleoside-phosphoramidite protected with the A group, w-A = an A-dNP appropriate to the wild-type sequence, B-dNP = deoxyribonucleoside-phosphoramidite protected with the B group, N-B = diluted solution of the four B-dNP's, **N-B** = concentrated solution of the four B-dNP's and S-A = concentrated solution of A-dG and A-dC-phosphoramidites.

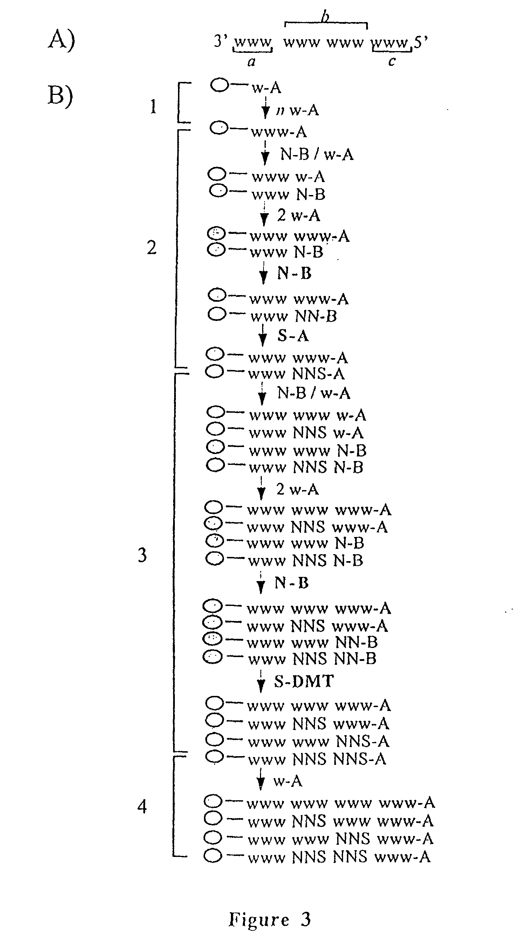

Figure 3. Protocol so-called "on-line mixing" for the generation of oligodeoxyribonucleotide libraries using the OCM method. The only difference with respect to the pre-addition protocol is that contamination is performed through the simultaneous delivery of the diluted mixture containing the four B-dNP's and the first A-dNP that defines the first nucleoside of the wild-type codon to be mutated. Where: ⊛ - Represents the CPG support, w = wild-type deoxyribonucleotide, A-dNP =deoxyribonucleoside-phosphoramidite protected with the A group, w-A = an A-dNP appropriate to the wild-type sequence, B-dNP = deoxyribonucleoside-phosphoramidite protected with the B group, N-B = diluted solution of the four B-dNP's, **N-B** = concentrated solution of the four B-dNP's and S-A = concentrated solution of A-dG and A-dC-phosphoramidites.

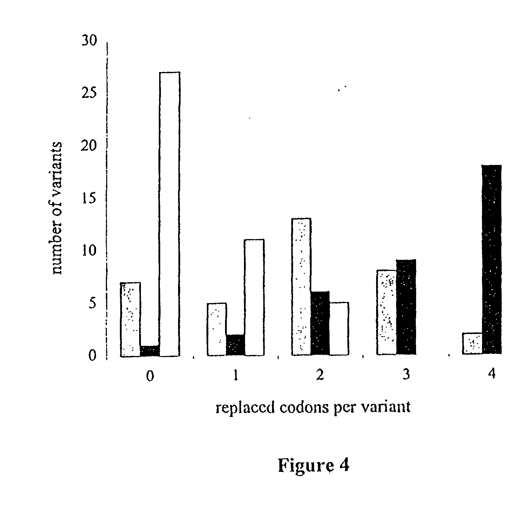

Figure 4. Combinatorial libraries of mutant oligodeoxyribonucleotides generated through the OCM method. Gray represents the distribution of mutants obtained in the first library ( $\alpha$ = 49.5%), black represents the distribution of mutants obtained in the second library ( $\alpha$ = 78.9%) and white represents the distribution of mutants obtained in the third library ($\alpha$ = 10.61%).

As mentioned in the above section, there are several methods of performing combinatorial mutagenesis through mixtures of oligodeoxyribonucleotides, in which some authors have described the use of trinucleotide mixtures to generate mutagenesis at codon instead of nucleotide level and report that it is possible to generate libraries that follow a predictable binomial distribution of mutants with advantages such as better exploration of the sequence space (by minimizing the problems associated with the degeneracy of the genetic code) and better distribution and frequency of variants. Nevertheless, an important limiting factor to these methods is that the reagents, particularly the trinucleotides, are not commercially available and furthermore their preparation involves considerable time and cost.

Furthermore, several authors describe codon-based mutagenesis methods that are based on the use of conventional dNP's, as is the case of the resin-splitting method in which the wild-type sequence of the oligonucleotide is assembled in a column, while in another one the mixture of mutant codons is synthesized using only conventional dNP's.

The resin-splitting method is highly manual, laborious, tedious and requires large amounts of CPG support and therefore large amounts of dNP's to be able to handle low levels of mutagenesis and large windows; otherwise this method is only viable for high levels of mutagenesis and small windows.

The inventors of the present invention propose a solution to these limiting factors that consists of an alternate method for the construction of binomial libraries of oligodeoxyribonucleotides mutagenized at a codon level, with clear advantages over the methods cited above, since using this method it is feasible to handle and control any level of mutagenesis and window size in a practically automated form.

The mutagenesis method of the present invention, that the inventors have called Orthogonal Combinatorial Mutagenesis (OCM) makes use of two sets of deoxyribonucleoside-phosphoramidites (dNP's) protected at the 5' hydroxyl with two orthogonal protecting groups. That is, they present opposing or contrary conditions of stability and remotion, as is the case of the 4,4'-dimethoxytrityl (DMT) group and the 9-fluorenylmethoxycarbonyl (Fmoc) group. The DMT group is labile in acid conditions and stable in alkaline conditions, while the Fmoc group is stable in acid conditions and labile in alkaline conditions. Furthermore, the derivatization of the 3' hydroxyl with the phosphoramidite function favors the incorporation of such dNP's in the automated synthesis of oligodeoxyribonucleotides.

The inventors propose the use of two sets of deoxyribonucleoside-phosphoramidites (dNP's) protected in their 5' hydroxyl with orthogonal protecting groups, for the construction of binomial libraries of oligodeoxyribonucleotides mutagenized at a codon level. This kind of oligodeoxyribonucleotides libraries mutagenized at a codon level follow a binomial distribution of variants according to the mutagenesis level. The invention comprises the following steps:

a) Sequentially couple on a solid support the deoxynucleoside-phosphoramidites (dNP's) protected with an A group (A-dNP's) in order to assemble the wild-type sequence corresponding to the 3' adjacent zone of the mutagenesis window to be explored in a determined gene;

b) starting a mutagenesis cycle consisting of the following stages:

1. couple the deoxynucleotide in the first position of the mutant codon through the addition of a mixture containing the dNP's protected with the B group (B-dNP's) in the 5' hydroxyl to the previously assembled sequence at an appropriate concentration that will permit the generation of a previously defined level of mutagenesis;

2. couple the deoxynucleotide in the first position of the wild-type codon to the oligodeoxyribonucleotide chains that did not react in the previous step through the addition of the appropriate A-dNP in accordance with the pattern of the wild-type sequence;

3. continue with the coupling of the second and third positions of both the mutant and wild-type codon without giving importance to the coupling order but conserving the following conditions: I) In the two subsequent couplings for the wild-type codon A-dNP's are used, in accordance with the pattern of the wild-type sequence; II) for the coupling of the second position of the mutant codon, a high concentration mixture containing B-dNP's is used; III) for the coupling of the third position of the mutant codon, a high concentration mixture containing dNP's protected with group B or A is used.

c) optionally, the mutagenesis cycle described in paragraph b) is repeated as many times as necessary in order to conclude the number of codons to be explored;

d) having finished the mutagenesis cycles, synthesize the wild-type sequence corresponding to the 5' zone adjacent to the mutagenesis window using the appropriate A-dNP's according to the wild-type sequence.

That is, in the Orthogonal Combinatorial Mutagenesis method, illustrated in figure 2, the wild-type sequence is assembled with A-dNP's, while for the assembly of the first and second position of the mutant codons, dNP's protected with a group orthogonal to the one used in the wild-type sequence, called B-dNP's, are used. Particularly, for the assembly of the third position of the mutant codons, dNP's protected with either the A group or the B group can be used indistinctly. It is important to mention that one important characteristic of the method of this invention is that the A and B protecting groups must exhibit orthogonal characteristics of stability and remotion between themselves. This characteristic favors the coupling of the dNP's, in a combined way, in order to synthesize the wild-type codon and the mutant codons.

There are several protecting groups that have been used for the protection of the 5' hydroxyl of the dNP's, among which we could mention: 4,4'-dimethoxytrityl (DMT), 4-monomethoxytrityl (MMT), terbutyldimethylsilyl (TBDMS), terbutyldiphenylsilyl (TBDPS), 9-fluorenylmethoxycarbonyl (Fmoc), levulinyl (Lev) and 2-dansylethoxycarbonyl, among others. The DMT group is the most commonly used for the protection of the dNP's used in the preparation of normal oligodeoxyribonucleotides and for this reason in this invention the inventors have called it a conventional group. This group is labile in weak acid conditions. The DMT-dNP's are commercially available.

The Fmoc group has several advantages over other protecting groups as orthogonal to DMT, because it is highly regio-selective towards the primary hydroxyl of the nucleosides, it is commercially available and is quickly removed with a weak alkaline treatment. Moreover, although the Fmoc-dNP's are not currently commercially available, their synthesis is relatively simple and proceeds with high yields.

The inventors propose the use of the DMT and Fmoc groups for protecting the dNP's used in the construction of binomial libraries of mutant oligodeoxyribonucleotides with the method of the present invention, given the characteristics of orthogonality among them and that it is not necessary to modify the standard synthesis protocols recommended by the manufacturer of the equipment conventionally used for oligodeoxyribonucleotide synthesis.

The couplings of the DMT-dNP's and Fmoc-dNP's were performed in an automatic DNA synthesizer using a normal synthesis protocol such as the one recommended by the manufacturer. This protocol includes the following steps for each coupling: 1) hydrolysis of the DMT group with an acid solution, for example, 2% dichloracetic acid in dichloromethane or hydrolysis of the Fmoc with an alkaline solution, for example DBU 0.1 M in acetonitrile; 2) coupling of the DMT-dNP or Fmoc-dNP with the growing oligonucleotide anchored to the solid support in the presence of tetrazole; 3) capping the 5' hydroxyls that did not react by acetylation and 4) oxidation of the recently formed phosphite triester to a more stable phosphate triester.

The creation of a mutagenesis strategy at a codon level that will generate binomial libraries of mutant oligodeoxyribonucleotides, using reagents that can be easily synthesized or commercially available, and in a practically automated way, may be a valuable tool for the study of the structure-function relation of proteins and their engineering. By means of this strategy, few amino acid replacements can be generated per mutant protein making this method suitable to research the individual importance of each of the wild-type amino acids to the function of the protein and at the same time avoid its functional destruction in order to improve, through in vitro evolution, a countless number of proteins with present and/or future commercial application, as for example the enzymes known as subtilisins, that have been modified to resist extreme temperature and pH conditions, which gives them advantages for use in biodegradable detergents.

Another way of performing the method for constructing binomial libraries in a simplified way, is described in Figure 3 and consists of modifying the mutagenesis cycle presented in paragraph b), carrying out steps 1 and 2 in just one step, through the simultaneous addition of a mixture of B-dNP's for the assembly of the first position of the mutant codon and the A-dNP corresponding to the wild-type sequence of the codon to be substituted. In this way, it is possible to reduce the number of steps in the mutagenesis cycle and the method therefore becomes more simple and rapid.

One way preferred by the inventors to perform the method for the construction of binomial libraries consists in carrying out the coupling of the third position of the mutant codon, by means of a high concentration mixture containing the dNP's protected with an A group.

This alternative makes possible, on the one hand, to ensure that the coupling of the third position of the mutant codons takes place in a high yield given the high concentration of the mixture and, on the other, on concluding the coupling of the mutant codons, all the sequences (wild-type and mutant codons) bear the same protecting group, that is A, and therefore at the end, it is necessary only one remotion step.

The method of the present invention preferably uses DMT as protecting group A and Fmoc as protecting group B. This option consists of using dNP's protected with the DMT group for the coupling of the codons in accordance with the pattern of the wild-type sequence and dNP's protected with the Fmoc group for the coupling of the mutant codons. This has certain advantages because, the reagents that are consumed in greater proportion are the DMT-dNP's which are currently commercially available. However, if in the future, commercial interest in the Fmoc-dNP's grows and these are commercially produced, the decision to use DMT as A group and F-moc as B group could be inverted in terms of criteria such as costs.

Another relevant point in the construction of binomial libraries using the method of the present invention consists in the flexibility of the mutagenesis window, since the number of codons to be explored can vary from a minimum of two codons to the maximum permitted by the particular characteristics of the synthesis equipment and the economics of the experiment.

At present, the available equipment permits synthesis of oligodeoxyribonucleotides of up to 100 nucleotides, thus discounting 10 base pairs for each of the ends adjacent to the window. This will make possible to handle a mutagenesis window of up to 80 nucleotides (approximately 27 codons).

It is obvious to an expert in the state of the art that as new equipments or new chemical systems permit the synthesis of longer oligodeoxyribonucleotides, the mutagenesis window may grow in the same proportion.

Another important aspect of the method of the present invention is flexibility with respect to the handling of the proportions of the dNP's used in the assembly of the mutant codons during the mutagenesis cycles. This means that the dNP's of adenine, guanine, cytosine and thymine, used for the coupling of the mutant codons, can be present in equimolar or in different proportions, which permits bias to the distribution of mutant codons towards a desired subset of coded amino acids. Furthermore, the number of dNP's present in the mixtures may be also variable. They may contain from 1 to the 4 dNP's. Similarly, it should be mentioned that these conditions prevail for each of the couplings of the different positions of the mutant codon.

One of the important, determining aspects in the construction of binomial libraries of mutant oligodeoxyribonucleotides using the method of the present invention is that the concentration of the mixture of dNP's used for the coupling of the first position of the mutant codons, directs and defines the level of mutagenesis (•).

In this sense it is important to emphasize that one characteristic of the method of the present invention is having complete control of the mutagenesis level. Nevertheless, it is preferable to handle low levels of mutagenesis to preferably generate low multiplicity mutants. In these cases, it is necessary that the concentration of the mixture of protected dNP's used for the coupling of the first position of the mutant codons to be preferably handled at low levels, independently of the number of protected dNP's it contains and of the proportions between them.

Moreover, by means of this method it is feasible to perform the coupling of the mutant codons with an NNG/C combination, achieving in this way a distribution of variants equivalent to that obtained by the resin-splitting method where N represents an equimolar mixture of the 4 B-dNP's and G/C, an equimolar mixture of deoxyguanosine and deoxycytidine.

Similarly, it is also feasible to favor certain subgroups of amino acids using the appropriate combinations of B-dNP's in the coupling of the mutant codons. This is in accordance with the distribution table proposed by Arkin & Youvan (1992).

It is important to emphasize the flexibility in the handling of the mutagenesis levels of the method of the present invention and the fact that the library of oligodeoxyribonucleotides mutagenized at a codon level has a population that follows a predictable binomial distribution by means of the combinatorial analysis equation:

$$P = \frac{n!}{x! \, (n-x)!} \, \alpha^x (1-\alpha)^{n-x}$$

Where:

**P** represents the proportion of a certain type of mutants (simple, double or triple, etc.) in the population.

*n* is the size of the mutagenesis window (expressed as the number of codons to be explored).

*x* the type of mutant (No. 1 corresponds to a simple mutant, No. 2 to a double one, etc.).

α the level or rate of mutagenesis.

In view that the present method allows the easy production of low multiplicity mutants, in this particularly case the level of mutagenesis • will be low and will be determined by the concentration of the mixture of the protected dNP's used for the coupling of the first position of the mutant codons.

Furthermore, an additional advantage in the construction of mutant libraries by means of this method is that they can be constructed on just one support, since dNP's protected with orthogonal groups display a high coupling efficiency under conventional working conditions. Therefore it is highly feasible that the process of the present invention will be automated, representing a clear advantage in comparison with the resin-splitting method.

In order to illustrate the application of the method of the present invention for the construction of binomial libraries of oligodeoxyribonucleotides mutagenized at a codon level, using dNP's, some examples performed for the synthesis of some oligodeoxyribonucleotide libraries generated at different levels of mutagenesis with 2 different synthesis protocols are described below.

EXAMPLES

The present invention is described in the following examples with the purpose of illustrating it better, but of course without restricting its scope.

Example 1

This example illustrates the chemical synthesis of four Fmoc-dNP's.

The four Fmoc-dNP's corresponding to adenine, guanine, cytosine and thymine were synthesized by the procedure reported by Lehmann et al. as illustrated in Figure 1 with only minor changes. 9-fluorenylmethoxycarbonyl chloride (Fmoc-Cl) was added at room temperature instead of 0 °C to N-acyl-deoxyribonucleosides or thymidine previously dissolved in pyridine and the reaction took place in only 5 min. instead of 30 minutes. In all the cases, tiny amounts of 3' and 3'-5' byproducts were generated and were removed by flash column chromatography. The deoxyribonucleosides protected in their 5' hydroxyl with the Fmoc group (Fmoc- deoxyribonucleosides) were recovered in high yields (60-70%) using methanol gradients in dichloromethane for the elution process. The phosphitylation of the Fmoc-deoxyribonucleosides was performed with chloro (diisopropylamino)methoxyphosphine and diisopropylethylamine in tetrahydrofuran as disolvent in order to give the corresponding Fmoc-dNP's that were purified by chromatography using 5% pyridine in dichloromethane as eluent.

The results showed that the 4 Fmoc-dNP's were obtained with at least 90% purity (assessed by HPLC and $^{31}$P NMR analysis) in global yields of 50-70%. It should be mentioned that an important contribution to this protocol to increase the total yield of the compounds containing Fmoc was the elimination of the washing step with sodium bicarbonate. Instead the reaction was worked-up only with brine and water in the presence of pyridine in order to avoid hydrolysis of the compounds.

Example 2

This example illustrates the relative reactivity of the 4 Fmoc-dNP's.

In order to assess the relative reactivity of the 4 Fmoc-dNP's, an equimolar solution of the four Fmoc-dNP's at a total concentration of 100 mM (25 mM each) in anhydrous acetonitrile was used. This mixture was placed in the vial X of the synthesizer. Three dinucleotides with an XC sequence were synthesized using the conventional DMTd-$C^{bz}$-CPG support as starting material and the coupling protocol recommended by the manufacturer for β-cyanoethylphosphoramidites. The Fmoc group was removed with a 100 mM solution of DBU in acetonitrile for one minute and the dimers were sequentially submitted to demethylation with thiophenol for 1 hour and to complete de-protection with concentrated $NH_4OH$ for 12 hours at 55 °C. The three dimer mixtures were analyzed by reverse phase HPLC in a similar way to that reported by Ward and Juehne, using an analytical vydac ODS column (4.6 x 250 mm) and a linear gradient of 30 to 70% of buffer B in 20 min., where buffer B was a 10% solution of acetonitrile in water and buffer A was a 0.1 M triethylamonium acetate solution at pH 7. The flow rate was 1 ml/min. and detection was at 260 nm..

The relative reactivity of each of the four Fmoc-dNP's was calculated by HPLC calibration curves of each of the dinucleotides, obtaining a relative reactivity of 31.4%, 22.4%, 20.8% and 25.4% for dA, dC, dG and dT respectively. As can be appreciated, the four Fmoc-dNP's showed different reactivities and therefore in order to obtain a homogeneous distribution of the 32 variant codons it may be necessary to adjust their concentrations when performing the library construction, where a greater amount of G and C will be used due to their lesser reactivity with respect to the other bases.

Example 3

This example illustrates the reactivity of the four Fmoc-dNP's as compared with four DMT-dNP's.

In this case, two mixtures containing two Fmoc-dNP's and two DMT-dNP's in acetonitrile, were prepared with each component at a 25 mM concentration (FmocdA-Me-amidite + FmocdC-Me-amidite + DMTdG-Me-amidite + DMT-dT-Me-amidite and DMTdA-Me-amidite + DMTdC-Me-amidite + FmocdG-Me-amidite + FmocdT-Me-amidite). These solutions were used in a similar way to example 2. In each case, three dinucleotides with a XC sequence were also prepared and assessed by HPLC.

In these experiments a relative reactivity of 12.7%, 12.2%, 13.3%, 12.5%, 14.2%, 9.9%, 12.2% and 13.0% was determined for DMT-dA, DMT-dC, DMT-dG, DMT-dT, Fmoc-dA, Fmoc-dC, Fmoc-dG and Fmoc-dT respectively. As can be appreciated, the reactivity of the Fmoc-dNP's with respect to all the DMT-dNP's shows small non-significant differences. In this sense it was concluded that the mutagenesis level could be easily controlled using molar equivalents for the level of substitution desired.

Example 4

This example illustrates the correlation between the level of mutagenesis and the binomial distribution of mutant oligodeoxyribonucleotides obtained in two libraries generated with the mutagenesis protocol called pre-addition (figure 2) in this work.

Two oligodeoxyribonucleotide libraries with the sequence 5' TAG GAG GAT CCC CGG GTA CCG AGC TCG AAT TCA CTC GGA C 3' were synthesized at different mutagenesis levels using the pre-addition protocol. This protocol consists in coupling the first deoxynucleotide of the mutant codons before the first deoxynucleotide of the wild-type codon, using a diluted mixture of the four Fmoc protected dNP's.

In this case, a synthesizer, labelled as "I", was loaded with 0.1 M solutions of each DMT-dNP's in their respective vials and the X position was loaded with a 20 mM solution of the four Fmoc-dNP's (5 mM each) in the case of the first library and a 50 mM solution (12.5 mM each) for the second library. The other auxiliary reagents in the synthesis were the conventional ones. Another DNA synthesizer labelled as "II" was loaded in position 1 with a solution of the four Fmoc-dNP's at a total concentration of 100 mM (25 mM each) and position 2 with an equimolar solution of DMTdG-Me-amidite and DMTdC-Me-amidite at a total concentration of 100 mM. As with synthesizer "I", the auxiliary reagents in the synthesis were the same, except the trichloroacetic acid solution that was substituted for by a solution of DBU 100 mM in acetonitrile. The codons to be randomly substituted were the ones underlined. All the sequences in both synthesizers were programmed to remain tritylated. The synthesis of the libraries was started with the programming of the 5' AG(C**X**) TCG AAT TCA CTC GGA C 3'sequence in synthesizer "I", omitting the acetylation step during the addition of X. The synthesis column was immediately transferred to synthesizer "II" and the NG/C sequence was added to complete the first group of mutant codons. The procedure was then repeated three times with the appropriate codons to complete the whole mutagenesis window. Finally, the addition of the 5' TAG GAG GAT CCC CGG 3' sequence was programmed in synthesizer "I". The fully protected oligonucleotide still anchored to the CPG support, was submitted to detritylation, demethylation with thiophenol for 1 hour in order to remove the internucleotidic methyl groups and subsequently treatment with concentrated ammonium hydroxide to remove all the remaining protecting groups. The oligodeoxyribonucleotide libraries were purified in 15% polyacrylamide gels containing 8 M urea and were recovered in deionized water after being desalted with n-butanol.

Example 5

This example illustrates the synthesis of oligodeoxyribonucleotide libraries by means of the mutagenesis protocol termed "on-line mixing" displayed in figure 3. This protocol is performed by mixing in the delivery lines of the synthesizer the DMT-dNP that defines the first nucleotide of the wild-type codon to be substituted and the mixture of Fmoc-dNP's that define the first position of the mutant codons.

An oligodeoxyribonucleotide library with the 5' TAG GAG GAT CCC CGG GTA CCG AGC TCG AAT TCA CTC GGA C 3' sequence was synthesized in order to assess the mutagenesis protocol termed on-line mixing.

In order to construct the library, synthesizer I was loaded with individual 150 mM solutions of the four DMT-dNP's in their respective vials and vial **X** was loaded with a 50 mM solution of the four Fmoc-dNP's (12.5 mM each). Synthesizer II was loaded as in example 4. The coupling of this oligodeoxyribonucleotide library started with the synthesis of fragment 5' AG(CX) TCG AAT TCA CTC GGA C 3' in synthesizer I, programmed to remain tritylated. The bases in parentheses were simultaneously added to the synthesis column from vial C and X for this first codon. The column was immediately transferred to synthesizer II and the NG/C sequence was added. This mutagenesis cycle was repeated a further three times with a mixture of the appropriate DMT-dNP line that defines the first position of the codon to be mutated and the four Fmoc-dNP's contained in vial X. This oligodeoxyribonucleotide library was finished in the same way as the library in example 4 and deprotected and purified in the same way.

In order to assess the final distribution of the mutant oligodeoxyribonucleotides in each of the three libraries, each one was independently mixed with the primer oligonucleotide 5' GTCCGAGTGAATTCG 3' and subjected to extension with klenow polymerase and deoxyribonucleosidetriphosphates in order to generate libraries of mutant duplexes that were digested with the restriction enzymes EcoRI and BamH I, and bound to pUC18 plasmids and expressed in the E. Coli JM101 strain. The mutant colonies were sequenced by analyzing 42, 38 and 49 colonies for the first, second and third library respectively.

The analysis of the codon replacements in Figure 4 showed that all the libraries follow approximately a binomial distribution of variants according to the mutagenesis level, that is with high levels of mutagenesis high multiplicity mutants were preferably generated (many changes of codon per gene), while for low levels of mutagenesis low multiplicity mutants were preferably generated (few changes of codon per gene). These results clearly indicate that the mutagenesis method proposed is highly predictable and that it corresponds to the distribution of variants expected. The results are shown in Table I.

Table I

| Clone Genotype | 1st library $\alpha$ = 49.5% | | 2nd library $\alpha$ = 78.9% | | 3rd library $\alpha$ = 10.61 % | |
|---|---|---|---|---|---|---|
| | # | % | # | % | # | % |
| Wild | 7 | 16.7 | 1 | 2.6 | 27 | 55.1 |
| Simple mutants | 5 | 11.9 | 2 | 5.2 | 11 | 22.4 |
| Double mutants | 13 | 30.9 | 6 | 15.9 | 5 | 10.2 |
| Triple mutants | 8 | 19.0 | 9 | 23.7 | 0 | 0 |
| Quadruple mutants | 2 | 4.8 | 18 | 47.4 | 0 | 0 |
| Deletions | 5 | 11.9 | 1 | 2.6 | 4 | 8.2 |
| Insertions | 2 | 4.8 | 1 | 2.6 | 2 | 4.1 |
| Total | 42 | 100 | 38 | 100 | 49 | 100 |

REFERENCIAS

Hermes, J. D., Parekh, S.M., Blacklow, S.C., Köster, H. & Knowles, J.R. (1989). A reliable method for random mutagenesis: the generation of mutant libraries using spiked oligodeoxyribonucleotide primers. Gene 84, 143-151.

Lehtovaara, P.M., Koivula, A.K., Bamford, J. & Knowles, J.K.C. (1988). A new method for random mutagenesis of complete genes: enzymatic generation of mutant libraries in vitro. Prot. Eng. 2, 63-68.

Sondek, J. & Shortle, D. (1992). A general strategy for random insertion and substitution mutagenesis: substoichiometric coupling of trinucleotides phosphoramidites. Proc. Natl. Acad. Sci. 89, 3581-3585.

Botstein, D. & Shortle, D. (1985). Strategies and applications of in vitro mutagenesis, Science 229, 1193-1201.

Myers, R.M., Lerman, L.S., & Maniatis, T. (1985). A general method for saturation mutagenesis of cloned DNA fragments, Science 229, 242-247.

Dunn, I.S., Cowan R. & Jennings P.A. (!988). Improved peptide function from random mutagenesis over short. Protein Eng. 2, 283 - 291.

Kadonaga, J.T. & Knowles, J.R. (1985). A simple and efficient method for chemical mutagenesis of DNA. Nucleic Acids Res. 13, 1733-1745.

Ner, S.S., Goodin, D.B. & Smith M. (1988) Laboratory Methods A Simple and Efficient Procedure for Generating Random Point Mutations and for Codon Replacements Using Mixed Oligodeoxynucleotides. DNA 7, 127 - 134.

Sirotkin, K. (1986). Advantages to mutagenesis techniques generating populations containing the complete spectrum of single codon changes. J. Theor. Biol. 123, 261-279.

Cormack B.P. & Struhlt K. (1993). Regional Codon Randomization: Defining a TATA-Binding Protein Surface Required for RNA Polymerase III Transcription. Science 262, 244 - 248.

Hooft van Huijsduijnen, R.A.M., Ayala G. & DeLamarter J.F. (1992). A means to reduce the complexity of oligonucleotides encoding degenerate peptides. Nucleic Acids Research 20, 919.

Arkin, A.P. & Yuovan D.C. (1992). Optimizing nucleotide mixtures to encode specific subsets of aminoacids for semi-random mutagenesis. Bio/Tech. 10, 297-300.

Reeve M.A. & Fuller C.W. (1995). A novel thermostable polymerase for DNA sequencing. Nature, 375, 796 - 798.

Del Río, G. Osuna, J. & Soberón, X. (1994). Combinatorial libraries of proteins: analysis of efficiency of mutagenesis techniques. BioTechniques 17, 1132-1139.

Virnekäs, B. & Moroney, S.E. (1994). Trinucleotide phosphoramidites: ideal reagents for the synthesis of mixed oligonucleotides for random mutagenesis. Nucleic Acids Res. 22, 5600-5607.

Ono, A., Matsuda, A., Zhao, J. & Santi, D.V. (1995). The synthesis of blocked-triplet-phosphoramidites and their use in mutagenesis. Nucleic Acids Res. 23, 4677-4682.

Lyttle, M.H. Napolitano, E.W., Calio, B.L., & Kauvar, L.M. (1995). Mutagenesis using trinucleotide $\beta$-cyanoethyl phosphoramidites. Nucleic Acid Res. 19, 274-280.

Kayushin, A.L., Korostelava, M.D., Miroshnikov, A.I., Kosch, W., Zubov, D. & Piel, N. (1996). A convenient approach to the synthesis of trinucleotide phosphoramidites-synthons for the generation of oligonucleotide/peptide libraries. Nucleic Acids Res. 24, 3748-3755.

## Claims

1. Method for the construction of binomial libraries of oligodeoxyribonucleotides mutagenized at a codon level, using two sets of deoxynucleoside-phosphoramidites (dNP's) protected in their 5' hydroxyl with two different groups, A and B, that are chemically orthogonal to one another, and comprises the following steps:

   a) sequentially coupling on a solid support the deoxyribonucleoside-phosphoramidites protected in their 5' hydroxyl with an A group (A-dNP's) in order to assemble the wild-type sequence corresponding to the 3' adjacent zone of the mutagenesis window to be explored in a determined gene;
   b) to begin a mutagenesis cycle comprising the following stages:

      1. to couple to the previously assembled sequence, the deoxyribonucleotides of the 1st position of the mutant codons by means of the addition of a mixture containing dNP's protected in their 5' hydroxyl with the B group (B-dNP's). This mixture must be used at a concentration that ensures a previously defined level of mutagenesis calculated through the equation of binomial distribution. This concentration will always be lower than that used for A-dNP's;

2. couple to the oligodeoxyribonucleotide chains that did not react in the previous step, the deoxyribonucleotide of the first position of the wild-type codon by means of the addition of the A-dNP, according to the pattern of the wild-type sequence;

3. continue with the coupling of the 2$^{nd}$ and 3$^{rd}$ position of both the mutant and the wild-type codon, without giving importance to the coupling order but conserving only the following conditions: I) In the two subsequent couplings A-dNP's are used for the wild-type codon according to the pattern of the wild-type sequence; II) for the coupling of the 2$^{nd}$ position of the mutant codon, a mixture containing B-dNP's at a concentration that ensures the coupling of a B-dNP in most of the molecules carrying a B-dNP in the prior position is used; III) for the coupling of the 3$^{rd}$ position of the mutant codon, a mixture containing dNP's protected with the B or A group at a concentration that ensures the coupling of an A or B-dNP in most of the molecules carrying a B-dNP in the prior position is used.

c) the mutagenesis cycle described in paragraph b) may be repeated optionally as often as necessary to conclude the number of codons to be explored;

d) when the mutagenesis cycles have been completed, synthesize the wild-type sequence corresponding to the 5' zone adjacent to the mutagenesis window using A-dNP's appropriate to the wild-type sequence.

2. Method for the construction of binomial libraries in accordance with claim 1, wherein the mutagenesis cycle of paragraph b) steps 1 and 2 takes place in just one step through the simultaneous addition of the A-dNP defined by the wild-type sequence, in order to assemble the 1$^{st}$ position of the wild-type codon and the mixture of B-dNP's that define the 1$^{st}$ position of the mutant codons.

3. Method for the construction of binomial libraries in accordance with either claim 1 or 2, wherein the coupling of the 3$^{rd}$ position of the mutant codon is performed by means of a mixture containing dNP's that are protected with an A group.

4. Method for the construction of binomial libraries in accordance with claim 1 or 2, wherein the protecting groups that are orthogonal to one another are 4,4'-dimethoxytrityl (DMT) and 9-fluorenylmethoxycarbonyl (Fmoc).

5. Method for the construction of binomial libraries in accordance with claim 4, wherein the A protecting group is 4,4'-dimethoxytrityl (DMT) and the B protecting group is 9-fluorenylmethoxycarbonyl (Fmoc).

6. Method for the construction of binomial libraries in accordance with either claim 1 or 2, wherein the mutagenesis window is defined trough sequential repetition of n mutagenesis cycles to conclude n codons to be explored.

7. Method for the construction of binomial libraries in accordance with either claim 1 or 2, wherein the mixture containing the B-dNP's used in step 1 of the mutagenesis cycle described in paragraph b) contains from one to four B-dNP's, independent of the proportions between one another.

8. Method for the construction of binomial libraries in accordance with claim 7, wherein the B-dNP's of the mixture are present in equimolar proportions,

9. Method for the construction of binomial libraries in accordance with claim 7, wherein the B-dNP's of the mixture are deoxyribonucleoside-phosphoramidites of adenine, guanine, cytosine and thymine, properly protected in the 5' hydroxyl with a B protecting group.

10. Method for the construction of binomial libraries in accordance with either claim 1 or 2, wherein the mixtures containing the dNP's used for the coupling of the 2$^{nd}$ and 3$^{rd}$ position of the mutant codon of the mutagenesis cycle described in paragraph b) contains from one to four dNP's protected in the 5' hydroxyl, independent of the proportions between them.

11. Method for the construction of binomial libraries in accordance with claim 10, wherein the dNP's of the mixture used for the coupling of the 2$^{nd}$ and 3$^{rd}$ position of the mutant codon are present in equimolar proportions.

12. Method for the construction of binomial libraries in accordance with claim 10, wherein the dNP's are deoxyribonucleoside-phosphoramidites of adenine, guanine, cytosine and thymine, properly protected in the 5' hydroxyl with a protecting group.

**13.** Method for the construction of binomial libraries in accordance with claim 10, wherein the mixture of the dNP's used for the coupling of the 3$^{rd}$ position of the mutant codon of the mutagenesis cycle of paragraph b) contains dNP's of only guanine and cytosine.

**14.** Method for the construction of binomial libraries in accordance with claims 1 or 2, wherein the concentration of the mixture of the dNP's protected in the 5' hydroxyl with group B for the coupling of the 1$^{st}$ position of the mutant codon and the mutagenesis level are directly proportional.

**15.** Method for the construction of binomial libraries in accordance with claim 14, wherein the level of mutagenesis and the multiplicity of mutants generated are directly proportional.

**16.** Method for the construction of binomial libraries in accordance with either claim 1 or 2, wherein the use of only one synthesis column and the combined use of orthogonal protecting groups favour its automation.

**Figure 1**

Figure 2

Figure 3

Figure 4

## SEQUENCE LISTING

<110> UNIVERSIDAD NACIONAL AUTONOMA DE MEXICO
SOBERÓN MAINERO, Francisco X

<120> Method for the construction of binomial libraries of
oligodeoxyribonucleotides, mutagenized at a codon level using
deoxyribonucleoside-phosphoramidites.

<130> monomeros PCT español

<140> PCT/MX00/00047
<141> 2000-11-15

<150> MX9910476
<151> 1999-11-15

<160> 1

<170> PatentIn Ver. 2.1

<210> 1
<211> 40
<212> DNA
<213> Artificial Sequence

<220>
<223> Description of Artificial Sequence: Synthesized

<400> 1
ctgcgagtga attcgagctc ggtacccggg gatcctccta                40